# FASCICULE DE BREVET EUROPEEN

(11) **EP 3 054 964 B1**
(45) Date de publication et mention de la délivrance du brevet: **03.06.2020**
(21) Numéro de dépôt: 14729667.7
(22) Date de dépôt: 13.06.2014
(51) Int. Cl.: A61K 36/81, A61P 35/00, A61P 27/02, A61P 9/00, A61P 9/10, A61P 29/00, A61P 3/00

(54) **PROCEDE D'OBTENTION D'UN EXTRAIT DE PLANTE ET COMPOSITIONS ASSOCIEES**
VERFAHREN ZUR HERSTELLUNG EINES PFLANZENEXTRAKTS UND ENTSPRECHENDE ZUSAMMENSETZUNGEN
METHOD FOR PRODUCING A PLANT EXTRACT AND ASSOCIATED COMPOSITIONS

(30) Priorité: 17.06.2013 FR 1355652
(43) Date de publication de la demande: 17.08.2016
(73) Titulaire: Ethnodyne, 75008 Paris (FR); Centre National de la Recherche Scientifique (C.N.R.S.), 75016 Paris (FR)
(72) Inventeur: RABHI, Chérif, F-91220 Bretigny Sur Orge (FR); CARIEL, Léon, 75005 Paris (FR); OUAZZANI, Jamal, F-91300 Massy (FR); ARCILE, Guillaume, F-91940 Les Ulis (FR)
(74) Mandataire: Casalonga
(86) Numéro de dépôt international: PCT/EP2014/062311
(87) Numéro de publication internationale: WO 2014/202469

(56) Documents cités:
- WO-A1-2011/150312
- US-A1- 2007 122 494
- US-A1- 2007 122 497
- PREETI TIWARI ET AL: "DIURETIC ACTIVITY OF ASHWAGA PREPARED BY TRADITIO A MODER METHODS I EXPERIME RATS", PHARMACOLOGY ONLINE, vol. 1, 1 août 2011 (2011-08-01), pages 740-747, XP055086859,
- JAGDISH MANWAR ET AL: "Comparative antioxidant potential of Withania somnifera based herbal formulation prepared by traditional and non-traditional fermentation processes", INTEGRATIVE MEDICINE RESEARCH, vol. 2, no. 2, 18 avril 2013 (2013-04-18), pages 56-61, XP055086855, ISSN: 2213-4220, DOI: 10.1016/j.imr.2013.04.002
- ARUN RASHEED ET AL: "Formulation, standardization and pharmacological evaluation of a poly herbal traditional remedy -Ashwagandharishtam", ORIENTAL PHARMACY AND EXPERIMENTAL MEDICINE, vol. 12, no. 1, 12 janvier 2012 (2012-01-12), pages 51-58, XP055086857, ISSN: 1598-2386, DOI: 10.1007/s13596-011-0050-2
- PETER G. MWITARI ET AL: "Antimicrobial Activity and Probable Mechanisms of Action of Medicinal Plants of Kenya: Withania somnifera, Warbugia ugandensis, Prunus africana and Plectrunthus barbatus", PLOS ONE, vol. 8, no. 6, 13 juin 2013 (2013-06-13), page e65619, XP055086845, DOI: 10.1371/journal.pone.0065619
- DATABASE MEDLINE [Online] US NATIONAL LIBRARY OF MEDICINE (NLM), BETHESDA, MD, US; 2002, PRAKASH JAI ET AL: "Withania somnifera root extract prevents DMBA-induced squamous cell carcinoma of skin in Swiss albino mice.", XP002716500, Database accession no. NLM12235655 & NUTRITION AND CANCER 2002, vol. 42, no. 1, 2002, pages 91-97, ISSN: 0163-5581
- LAKSHMI-CHANDRA MISHRA ET AL: "SCIENTIFIC BASIS FOR THE THERAPEUTIC USE OF WITHANIA SOMNIFERA (ASHWAGANDHA): A REVIEW", ALTERNATIVE MEDICINE REVIEW, THORNE RESEARCH INC., SANDPOINT, US, vol. 5, no. 4, 1 août 2000 (2000-08-01), pages 334-346, XP002216001, ISSN: 1089-5159
- "Withania somnifera", New Crop Resource Online Program of Purdue University , 4 août 2004 (2004-08-04), XP002716497, Extrait de l'Internet: URL:http://www.hort.purdue.edu/newcrop/cro pfactsheets/asgandh.html [extrait le 2013-11-13]
- "Ashwagandha", Memorial Sloan-Kettering Cancer Center , 21 décembre 2011 (2011-12-21), XP002716501, Extrait de l'Internet: URL:http://www.mskcc.org/cancer-care/herb/ ashwagandha [extrait le 2013-11-13]
- "Emblica officinalis Gaertn", New Crop Resource Online Program of Purdue University , 1 avril 2002 (2002-04-01), XP002716498, Extrait de l'Internet: URL:http://www.hort.purdue.edu/newcrop/par mar/07.html [extrait le 2013-11-13]
- "Bacopa monnieri", Wikipedia , 10 juin 2013 (2013-06-10), XP002716499, Extrait de l'Internet: URL:http://en.wikipedia.org/w/index.php?ti tle=Bacopa_monnieri&oldid=559261802 [extrait le 2013-11-13]

## Description

L'invention concerne un procédé d'obtention, à partir d'un extrait de plante, d'une composition pour aider au traitement ou à la prévention de désordres ou pathologies liés à la néo-vascularisation.

L'angiogenèse ou vascularisation est un phénomène physiologique hautement régulé par lequel de nouveaux vaisseaux sanguins sont produits dans un tissu ou un organe. L'angiogenèse dite normale est activée dans des situations de cicatrisation de plaies ou dans le développement de l'environnement fœtal.

L'angiogenèse pathologique est activée dans le cas de désordres cellulaires ou métaboliques liés ou non à l'âge et va être responsable de maladies potentiellement très invalidantes. Parmi elles, plusieurs maladies oculaires dont la plus connue est la DMLA (Dégénérescence Maculaire liée à l'âge), la rétinopathie liée au diabète, et toute une série de kératites et de glaucomes associés à une vascularisation anarchique.

L'angiogenèse pathologique est également soupçonnée dans l'arthrite rhumatoïde, l'arthrose, la maladie de Crohn, l'athérosclérose, les différentes formes d'angiomes de l'enfance ou liés à des maladies héréditaires.

Enfin, l'angiogenèse est fondamentale dans le développement de tumeurs solides et des métastases. Les tumeurs dans lesquelles l'angiogenèse est importante comprennent également des tumeurs bénignes telles que neurinome de l'acoustique, le neurofibrome, le trachome et des granulomes pyogènes. La prévention de l'angiogenèse peut stopper la croissance de ces tumeurs et les dommages qui en résultent.

Un certain nombre de molécules sont connues pour avoir un effet inhibiteur sur la néo-angiogenèse, telles que la protamine, le tétrahydrocortisol, la fumagelline, les dérivés de l'acide ascorbique, les glycoprotéines animales et des facteurs cellulaires tel que l'interféron. Cependant, le manque d'efficacité de ces molécules, leur toxicité ou la difficulté d'administration, en particulier pour les facteurs protéiques, limitent leur intérêt. En outre, les utilisateurs sont de plus en plus sensibles à l'origine des produits ainsi que le mode d'administration et favorisent en particulier les produits d'origine naturelle.

Il est décrit, que les extraits de *Withania Somnifera, d'Emblica Officinalis* et de *Bacopa Monnieri,* présentent une activité anti-angiogénique. Cependant, les extraits de ces plantes ne sont pas utilisés comme tels, à cause d'une toxicité élevée liée à l'obtention des extraits et en particulier de l'extrait de *Withania Somnifera.*

De manière surprenante, la demanderesse a trouvé qu'en associant, dans un procédé à partir de plante *Withania somnifera,* une étape d'extraction et une étape de fermentation par un champignon filamenteux, on pouvait agir sur la toxicité des extraits.

L'invention a donc pour but de proposer un procédé d'obtention d'une composition non toxique à base d'extrait de *Withania Somnifera,* ayant un effet inhibiteur de la néo-vascularisation, ainsi que les compositions ainsi obtenues.

La présente invention se rapporte donc à un procédé d'obtention d'une composition, comprenant au moins les étapes suivantes :
- Réalisation d'un extrait *de Withania Somnifera*
- Fermentation dudit extrait par une mise en incubation de cet extrait avec un champignon filamenteux de la famille des *Cordycipitaceae* dans un milieu adapté.

L'invention porte également sur une composition susceptible d'être obtenue par le procédé de l'invention.

Un autre objet de l'invention et l'utilisation de cette composition comme complément alimentaire.

Un autre l'invention est l'utilisation de cette composition comme médicament.

D'autres objets, caractéristiques, aspects et avantages de l'invention apparaîtront encore plus clairement à la lecture de la description et des exemples qui suivent :
- La figure 1A représente une empreinte chromatographique de l'extrait issu de l'extraction de *Withania Somnifera* sans fermentation,
- La figure 1B représente une empreinte chromatographique de l'extrait issu de l'extraction de *Withania Somnifera* non frementé sans withanolides,
- La figure 2 représente une empreinte chromatographique de l'extrait issu de l'extraction de *Bacopa Monierri* sans fermentation,
- La figure 3 représente une empreinte chromatographique de l'extrait issu de l'extraction *d'Emblica Officinalis* sans fermentation,
- La figure 4 représente une empreinte chromatographique de l'extrait issu de l'extraction de *Calendula Officinalis* sans fermentation,
- La figure 5 représente une empreinte chromatographique de l'extrait commercial de *Punica Granatum* sans fermentation,
- La figure 6 représente une empreinte chromatographique de l'extrait commercial de *Curcuma Longa* sans fermentation,
- La figure 7 représente une empreinte chromatographique de l'extrait issu de l'extraction de *Piper Longum* sans fermentation,
- La figure 8 représente une empreinte chromatographique de la composition initiale avant l'étape de fermentation.
- La figure 9 représente une empreinte chromatographique de la composition selon l'exemple 8 après 7 jours de fermentation de cette composition en présence de la souche *Beauveria bassiana* ATCC 7159,
- La figure 10 représente une empreinte chromatographique de la composition selon l'exemple 10,
- La figure 11 représente une empreinte chromatographique de la composition selon l'exemple 11,
- La figure 12 représente une empreinte chromatographique de la composition selon l'exemple 12,
- Les figures 13A, 13B 13C et 13D représentent des photographies de la membrane chorioallantoique d'un embryon de poulet (CAM) à respectivement 0 et 24 heures après exposition à 40 µl de la composition de l'exemple 8.
- Les figures 14A, 14B 14C et 14D représentent des photographies de la membrane chorioallantoique d'un embryon de poulet (CAM) à respectivement 0, 24, 48 et 72 heures après exposition à 40 µl de la composition de l'exemple 9.

La plante *Withania Somnifera* est obtenue de l'Inde. La racine de cette plante est commercialisée par Alp Erbo (Marseille).

La réalisation des extraits est effectuée par tous moyens connus de l'homme du métier, tel qu'une macération dans des solutions aqueuses, alcooliques ou hydroalcooliques de poudres végétales issues d'une ou des différentes parties de la plante tel que la racine, les feuilles, la tige, les branches, le fruit ou les fleurs, mais également par décoction, par un fluide supercritique, ou subcritiques, par adsorption. (Indian J Pharm Sci. 2010 Sep-Oct; 72(5): 659-663), EP2138054, thèse de l'université de Toulouse, "Étude des procédés d'extraction et de purification de produits bioactifs à partir de plantes par couplage de techniques séparatives à basses et hautes pressions" soutenue par Petko Ivanov PENCHEV, 2010.

Le champignon filamenteux utilisé pour la fermentation est choisi parmi les champignons de la famille des *Cordycipitaceae.* De préférence, le champignon est choisi parmi les champignons appartenant au genre Beauveria. Plus préférentiellement, ledit champignon filamenteux est issu de la souche *Beauveria Bassina,* plus particulièrement la souche portant la référence ATCC 7159.

En effet, la souche de *Beauveria bassiana* présente des propriétés catalytiques avantageuses et n'est pas toxique pour l'homme. Cette souche est couramment utilisée dans l'agriculture dans la lutte biologique contre les insectes, mais également comme probiotique pour les animaux.

Ainsi, la fermentation contrôlée et notamment celle réalisée par la souche *Beauveria bassiana* ATCC 7159 (American Type Culture Collection) permet la détoxification de l'extrait de *Withania Somnifera* par une série de biocatalyses de différentes molécules contenues dans cet extrait et plus particulièrement, la famille chimique des withanolides aglyconées, principales responsables de la toxicité de l'extrait.

On entend par « fermentation », l'action de transformation de substances végétales sous l'action de microorganismes.

On entend par « milieu adapté » tous milieux connus de l'homme du métier permettant le développement de la biomasse fongique nécessaire à la fermentation. Des exemples de milieux adaptés sont le Sabouraud Dextrose Agar (SAB) (Gibco, France), Brain-Heart Infusion Agar (BHI) (Gibco, France), Malt Extra Broth (MEB) (Gibco, France), Yeast Malt extract (YM) (Gibco, France), Yeast Extract-Phosphate Médium (YEP) (Gibco, France), Dermatophyte Test Médium (DTM) (Gibco, France), Potato dextrose Agar and Broth (PDA, RDB) (Gibco, France), ou C-Medium . Préférentiellement, le milieu C de composition suivante est utilisé. Pour 1 litre d'eau, on ajoute 10 g de mélasse, 0.5 g of MgSO₄, 2 g of NaNO₃, 0.5 g of KCl and 0.02 g of FeSO₄ (composés obtenus chez Sigma, France).

On entend par "détoxification" l'élimination par le microorganisme des molécules potentiellement toxiques dans le milieu.

Selon une caractéristique, le procédé selon l'invention comporte une étape de filtration du milieu d'incubation ayant servi lors de l'étape de fermentation, afin d'éliminer le microorganisme utilisée lors de la fermentation. Cette étape de filtration peut être réalisée par décantation ou filtration mécanique par tous moyens connus de l'homme du métier tel que du papier filtre ou des filtres.

Selon une autre caractéristique, le procédé selon l'invention peut comporter une étape de stérilisation qui peut être réalisée par tous moyens connus de l'homme du métier, notamment par autoclave, par exemple à 120°C pendant 20 minutes, par ultra filtration ou par rayonnement.
De préférence, le milieu d'incubation après l'étape de fermentation, de filtration et de stérilisation, est ensuite soumis à une ultrafiltration pour obtenir la solution qui constitue l'extrait de plante ((Millipore, Applied Membranes), *Ultrafiltration de fluide alimentaire fortement colmatant: flux limite, flux critique et nettoyabilité d'une membrane polymère,* Ndéye Wemsy Diagne, Murielle Rabiller-Baudry, IUT Rennes, Université Rennes I, UMR CNRS 6226).

Dans un mode de réalisation préféré, le procédé de l'invention peut être mis en œuvre pour d'autres plantes que *Withania Somnifera.* En particulier, on pourra aussi produire des extraits *d'Emblica Officinalis,* provenant d'Inde et commercialisé par Infrag, Bengalore) de *Bacopa Monnieri* (Inde) commercialisé par Alp Erbo (Marseille), de *Punica Granatum* (Chine) (Société Shangai Brightol international Co, Ltd (Shanghai), de *Curcuma Longa* (Inde)(Omnipharm, Chambery), de *Piper Longum* (Thailande)(Omnipharm, Chambery), ou de *Calendula Officinalis* (Chine) (Société Shangai Brightol international Co, Ltd (Shanghai) selon le même mode opératoire).

Selon un autre mode de réalisation, le procédé de l'invention comprend l'étape d'ajouter à l'extrait de *Withania Somnifera,* un extrait *d'Emblica Officinalis* et un extrait de *Bacopa Monnieri* avant de réaliser la fermentation desdits extraits avec ledit champignon filamenteux dans un milieu adapté.

Selon ce mode de réalisation, le procédé comporte des étapes d'extraction indépendantes pour chaque extrait de plantes utilisé pour la réalisation de ladite préparation. C'est-à-dire que les extraits de plantes sont réalisés indépendamment des un des autres.

Un autre objet de l'invention porte sur la composition issue du procédé de l'invention.

Cette composition conforme à l'invention contient ainsi un extrait de *Withania Somnifera.* Elle peut également comprendre au moins un des extraits suivants : extraits *d'Emblica Officinalis,* de *Bacopa Monnieri,* de *Punica Granatum,* de *Curcuma Longa,* de *Piper Longum,* ou de *Calendula Officinalis.*

Avantageusement, cette composition comprend en poids entre 5 et 100 g/L de *Withania Somnifera,* de préférence 20 g/L Préférentiellement, cette composition comprend de plus un des extraits suivants exprimés en poids:
- entre 5 et 100 g/L de l*'Emblica Officinalis,* de préférence 15 g/L,
- entre 5 et 100 g/L de *Bacopa Monierri,* de préférence 15g/L,
- entre 5 et 50 g/L de *Punica Granatum,* de préférence 10g/L,
- entre 5 et 250 g/L de *Curcuma Longa*, de préférence 20g/L,
- entre 20 et 50 mg/L de *Piper Longum,* de préférence 30mg/L,
- entre 5 et 50 g/L de Calendula Officinalis, de préférence 10g/L.

De préférence, la composition conforme à l'invention comprend un extrait des plantes *Withania Somnifera, Emblica Officinali* et *Bacopa Monnieri* susceptible d'être obtenu par le procédé de l'invention.

La composition peut être sous forme liquide, gel, émulsion, solide ou injectable.

La composition selon l'invention est formulée pour une administration topique, ophtalmique, transdermique, orale, rectale ou parentérale.

L'homme de l'art dans le domaine de la galénique saura mettre en œuvre les différentes formes utiles pour l'administration des produits et/ou compléments de l'invention.

La composition selon l'invention peut en outre comporter des suspensions, des émulsions, des sirops contenant des diluants inertes classiquement utilisés, et éventuellement d'autres substances comme des produits mouillants, édulcorants, conservateurs, épaississants, colorants, ou toutes autres substances connues de l'homme du métier propre à une administration orale ou oculaire en particulier ((sorbate de sodium (E201) (Sigma-Aldrich), anthocyane (E163) (FBC Industries, USA), disulfite de sodium (E223) (Sigma-Aldrich), alpha-tocophérol (E307) (FBC Industries, USA).

La composition selon l'invention peut également comprendre des solvants ou autres excipients tels que l'eau, le propylène glycol, des huiles végétales ou d'autres solvants organiques convenables.

On entend par «excipient» tout composé qui n'interfère pas avec l'efficacité de l'activité biologique de la composition selon l'invention et qui n'est pas toxique pour l'hôte auquel il est administré.

Ladite composition selon l'invention peut également contenir des adjuvants, comme des agents mouillants, isotonisants, émulsifiants, des sels ou toutes autres substances connues de l'homme du métier pouvant être utilisées comme adjuvants (Polydimethylsiloxane, l'alcool polyvinylique (APV), les hydrogels (Carbopol), polyvinyl-pyrrolidone, hydroxypropylcellulose (HPC), poloxamère 188, EDTA, chlorobutanol)(Lubrizol, France, Dow Corning, USA).

Avantageusement, la composition selon l'invention peut comprendre d'autres substances dans la formulation dudit complément alimentaire ou médicament, telles que des vitamines, des sels minéraux, un vecteur pharmaceutiquement acceptable, des stabilisants, des antioxydants, ou toutes autres substances connues de l'homme du métier destinées à être intégrées dans un complément alimentaire ou médicament.

On entend par « vecteur pharmaceutiquement acceptable » tout vecteur qui n'interfère pas avec l'efficacité de l'activité biologique de la composition selon l'invention et qui n'est pas toxique pour l'hôte auquel il est administré.

Suite à ce procédé, la composition obtenue est utilisable chez un mammifère, et plus particulièrement un humain, pour aider au traitement ou à la prévention de désordres ou pathologies liés à la néo-vascularisation.

Ladite composition peut être ainsi utilisée sous la forme de complément alimentaire.

Par « complément alimentaire » on entend un produit renfermant ladite composition ayant pour objet de compléter l'alimentation en apportant des nutriments bénéfiques pour la santé selon la définition donnée par la directive européenne 2002/46/CE. Par exemple un complément alimentaire peut être une gélule ou un comprimé à avaler ou une poudre ou une petite ampoule à mélanger à un aliment et présentant des effets bénéfiques pour la santé.

Ladite composition peut également être utilisée comme médicament.

Par « médicament », on entend un produit renfermant une dose précise de ladite préparation selon la directive européenne 65/65/CE à savoir toute substance ou composition présentée comme possédant des propriétés curatives ou préventives à l'égard des maladies humaines ou animales. Par exemple, le médicament contenant ladite préparation aux doses thérapeutiques peut être administré par voie orale sous forme de gélule ou comprimé ou injecté par voie intra-vitréenne ou toutes autres voies permettant de conférer des effets bénéfiques.

La composition selon l'invention est utilisable pour aider au traitement ou à la prévention de désordres ou pathologies liés à la néo-vascularisation, tels que la DMLA, la rétinopathie liée au diabète, les kératites et les glaucomes associés à une vascularisation anarchique. Elle est également utile pour le traitement de l'arthrite rhumatoïde, l'arthrose, la maladie de Crohn, l'athérosclérose, les formes d'angiomes de l'enfance ou liés à des maladies héréditaires, pour le traitement du cancer, et plus particulièrement des tumeurs solides et des métastases ainsi que pour le traitement des tumeurs bénignes telles que neurinome de l'acoustique, le neurofibrome, le trachome et des granulomes pyogènes.

La dose journalière des compositions et/ou compléments selon l'invention peut varier selon les besoins et la gravité des symptômes du patient. Typiquement, la dose journalière se situe entre 10mg/mL et 300mg/mL de la solution après fermentation.

De préférence, la dose journalière pour un humain adulte est entre 30 et 100mg/mL de la solution après fermentation.

La présente invention est maintenant décrite à l'aide d'exemples de réalisation, uniquement illustratifs et nullement limitatifs de la portée de l'invention.

### Exemple 1 : Extrait de Withania Somnifera avant fermentation

L'extrait de *Withania Somnifera* (originaire du Sud de l'Asie, et provient de l'Inde) a été réalisé à partir d'une quantité de 650 g de racine (Alp Erbo, Marseille), dans un mélange de 8 litres de solvant hydroalcoolique, éthanol/eau (60:40).

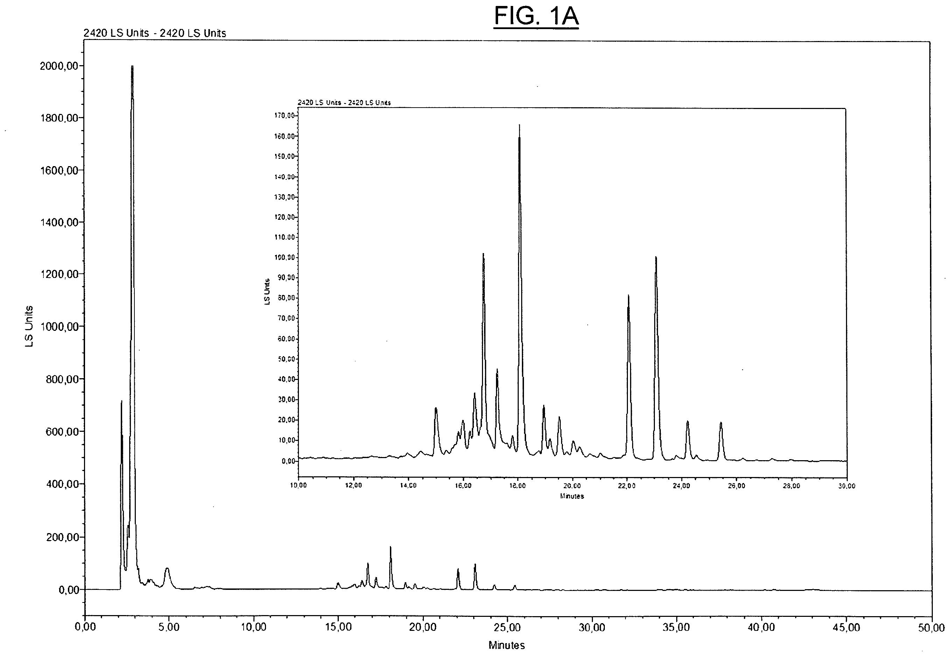

Il a été obtenu un extrait amorphe de 87g, soit un rendement de 13.4% qui contient des withanosides A et B, des withanosides, des sitoindosides (Figure 1A).

Afin d'éliminer les withanolides considérés comme toxiques, une extraction liquide/liquide avec du CH₂Cl₂ a été effectuée. Après rinçage et séchage de la phase organique, il a été obtenu : 81.5g d'une poudre amorphe soit un rendement de 94 %.

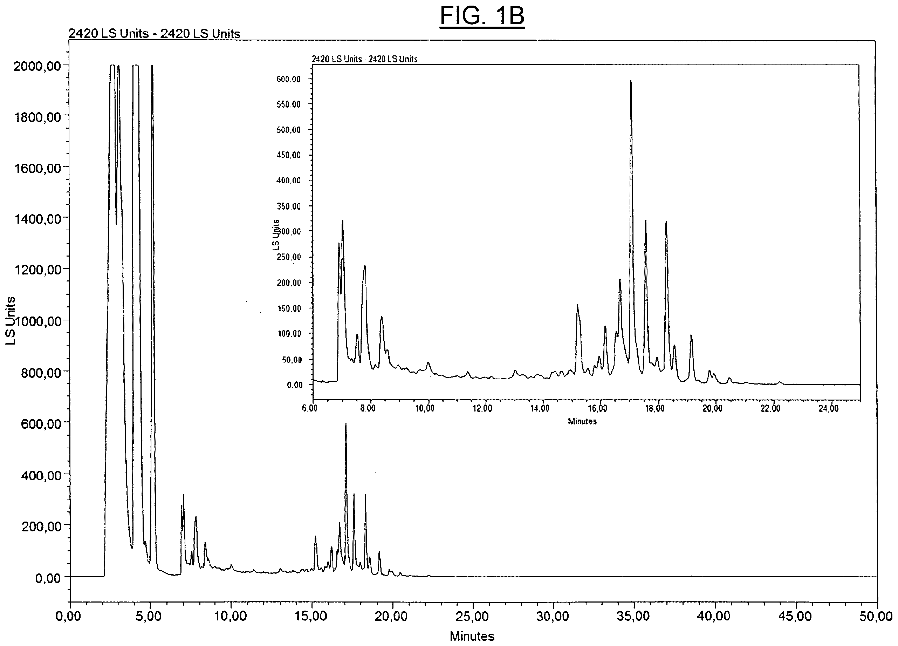

Cet extrait a été par la suite analysé puis identifié par des techniques de type HPLC, HPLC-MS et RMN afin d'obtenir une empreinte chromatographique de l'extrait de *Withania Somnifera* (figure 1B).

Les échantillons obtenus ont été analysé sur une chaîne HPLC analytique équipée d'une colonne en phase inverse de type Sunfire III C18 (4,6 x 150 mm) 3,5 µm (Waters), une chaîne HPLC Alliance® Waters W2695 munie d'un détecteur PDA Waters 2996. Ce système chromatographique est couplé à un détecteur évaporatif à diffusion de lumière (DEDL) Waters 2424. Le système HPLC est piloté par le logiciel Empower 2 (Waters).

Les solvants utilisés sont composés d'eau HPLC (milli Q) + 0.1% Acide Formique, d'acétonitrile (HPLC grade) + 0.1% Acide Formique. Le gradient standard utilisé est de 0 à 100% acétonitrile en 40 min + 10 min à 100% acétonitrile (durée totale 50 min). Le débit est de 0,7mL/min et le volume d'injection de 20 à 100 µL selon l'échantillon.

Pour la spectrométrie de Masse, les analyses HPLC-MS sont effectuées sur une chaîne HPLC Alliance® Waters couplée à un détecteur UV type PDA Waters 2998, un détecteur évaporatif à diffusion de lumière DEDL Waters 2420 et à un détecteur de masse Micromass® ZQ (Waters).

Les solvants sont de l'eau HPLC (milli Q) + 0.1% Acide Formique et de l'acétonitrile (HPLC grade) + 0.1% Acide Formique. Le gradient standard utilisé est de 0 à 100% acétonitrile en 40 min + 10 min à 100% acétonitrile (durée totale 50 min). Le débit est de 0,7mL/min et le volume d'injection est de 20 à 100 µL selon l'échantillon.

Les prélèvements de chaque composition pour analyse HPLC sont filtrés 0,45 microns (Ait-France, ref : SFNY 013045N), puis on injecte un volume de l'ordre de 50µl.

### Exemple 2: extrait de Bacopa Monierri avant fermentation

L'extraction de *Bacopa Monierri* a été réalisée à partir d'une quantité de l'ordre de 418 g dans un mélange de 5 litres de solvant hydroalcoolique, éthanol/eau (50:50).

Il a été obtenu un extrait amorphe de 77,1 g, soit un rendement de 18,4%, qui contient des bacosides A et B.

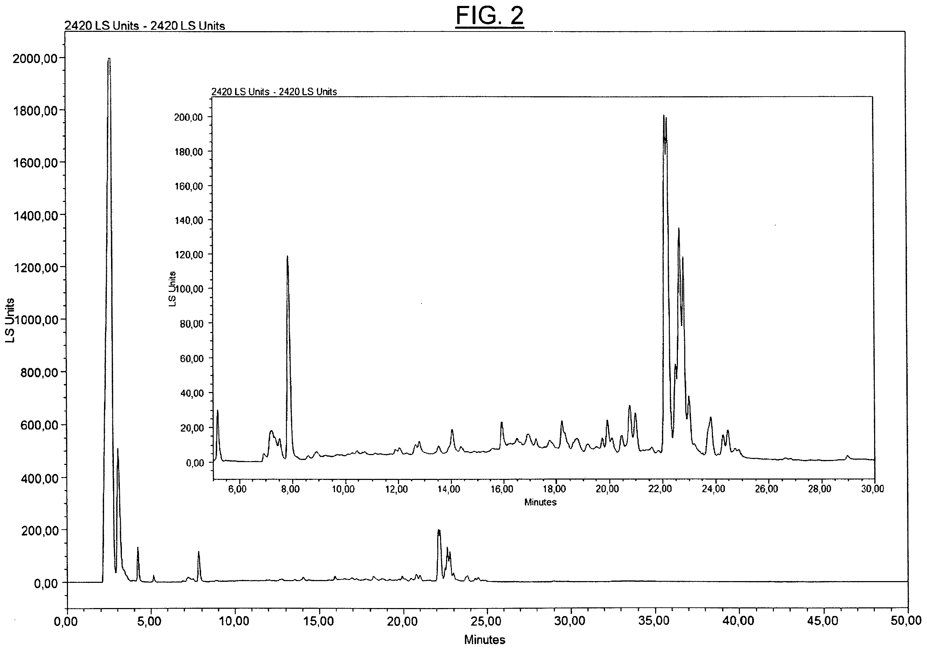

Cet extrait a été analysé puis identifié par des techniques de type HPLC, HPLC-MS et RMN afin d'obtenir une empreinte chromatographique de l'extrait de *Bacopa Monierri* (figure 2).

### Exemple 3 : Extrait d'Emblica Officinalis avant fermentation

L'extraction d'Emblica Officinalis a été réalisée sur une quantité de l'ordre de 512 g dans un mélange de 7 litres de solvant alcoolique composé d'éthanol à 100%.

Il a été obtenu un extrait amorphe de 166,5 g, soit un rendement de 32,5 %, qui contient des tannins galliques, de la vitamine C, de l'acide ellagique.

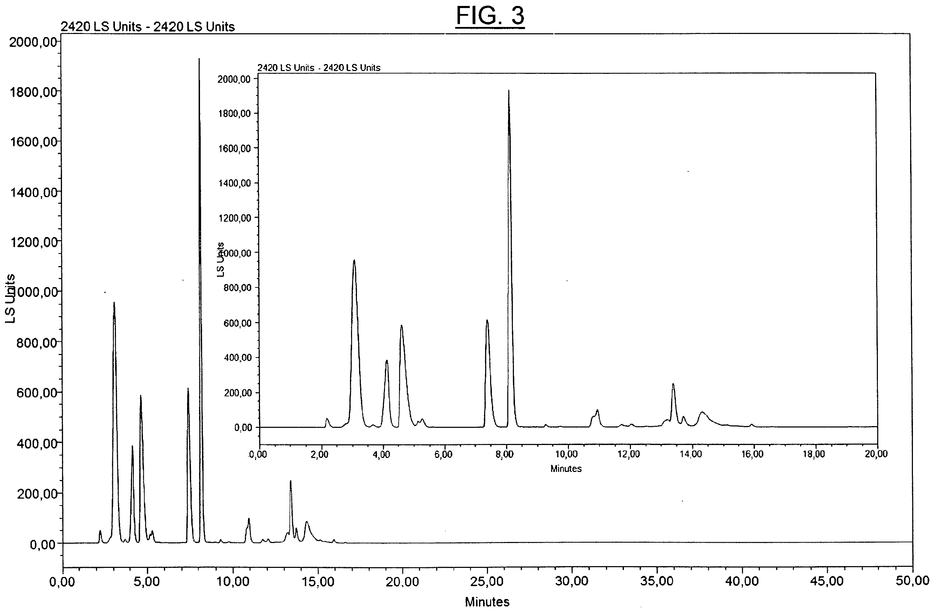

Cet extrait a été analysé puis identifié par des techniques de type HPLC, HPLC-MS et RMN afin d'obtenir une empreinte chromatographique de l'extrait d'Emblica Officinalis (figure 3).

### Exemple 4 : Extrait de Calendula Officinalis avant fermentation

L'extrait de *Calendula Officinalis* titré à 10% en lutéine et 0.9% en Zéaxanthine% a été obtenu auprès de la société Shangai Brightol international Co, (Shanghai).

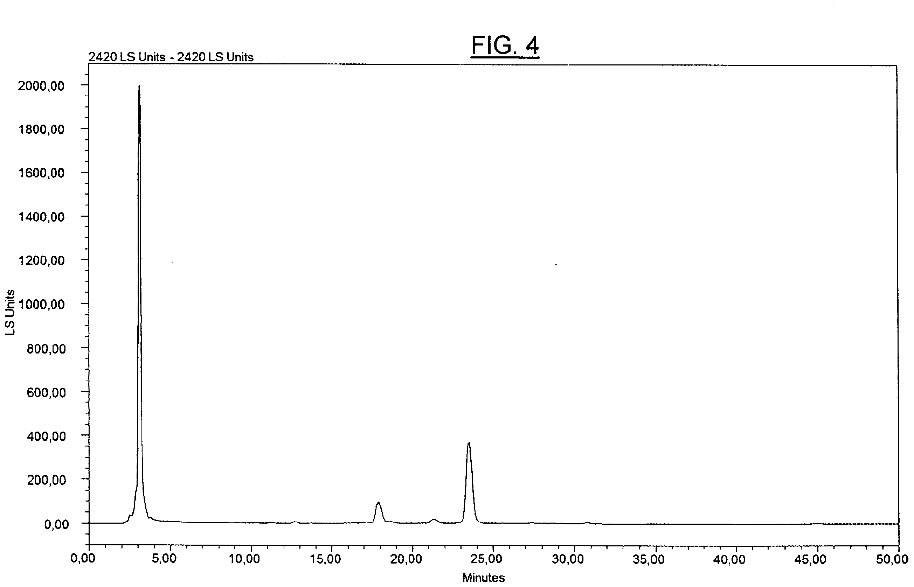

Cet extrait a été analysé puis identifié par des techniques de type HPLC, HPLC-MS et RMN afin d'obtenir une empreinte chromatographique de l'extrait de *Punica Granatum* (figure 4).

### Exemple 5 : Extrait de Punica Granatum avant fermentation

L'extrait de *Punica granatum* titré à 40% en acide ellagique a été obtenu auprès de la société Shangai Brightol international Co, Ltd (Shanghai).

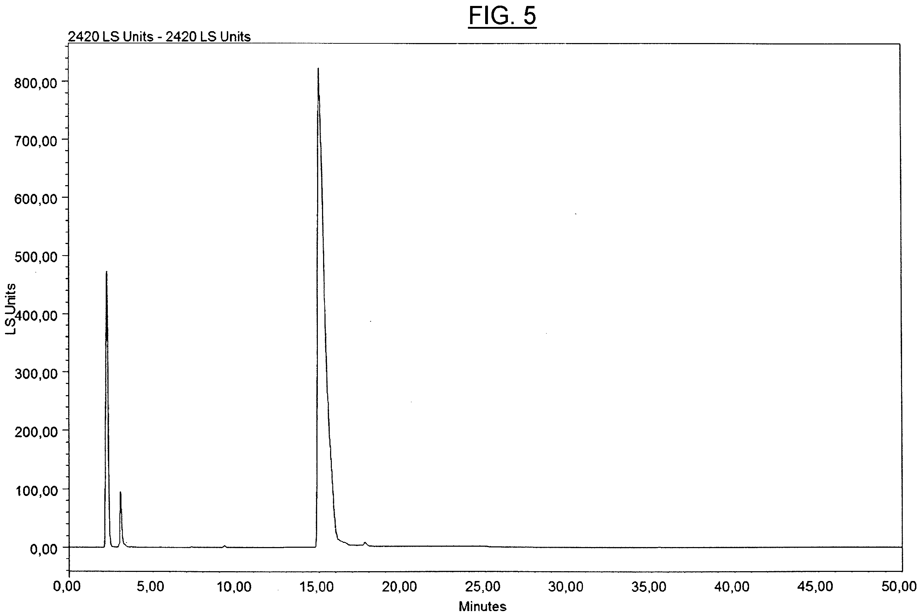

Cet extrait a été analysé puis identifié par des techniques de type HPLC, HPLC-MS et RMN afin d'obtenir une empreinte chromatographique de l'extrait de *Punica Granatum* (figure 5).

### Exemple 6 : Extrait de Curcuma Longa avant fermentation

L'extrait de rhizome de curcuma titré à 95% en curcumine provient de la société COOPER, Melun, (N° de lot : 120 105 B, CE n°207-280-5).

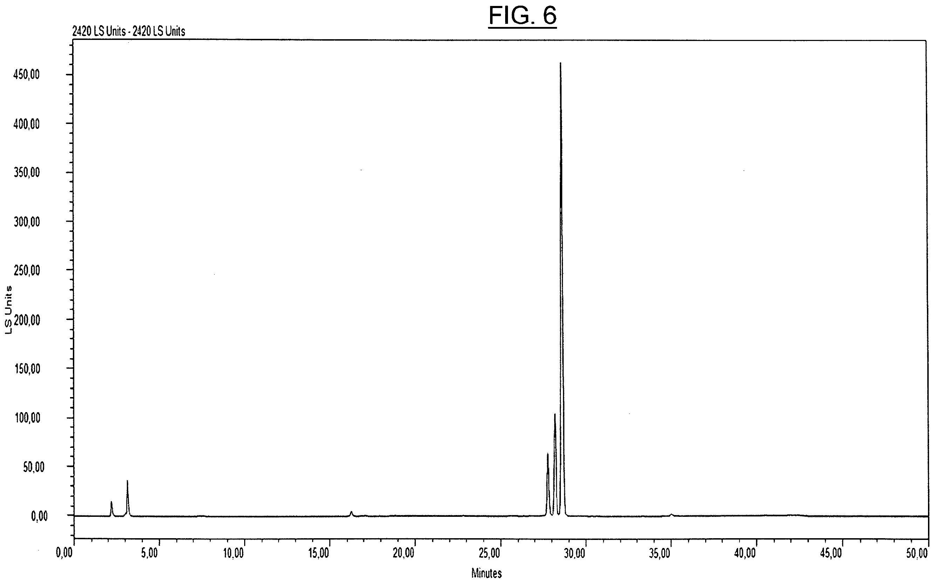

Cet extrait a été analysé puis identifié par des techniques de type HPLC, HPLC-MS et RMN afin d'obtenir une empreinte chromatographique de l'extrait de *Curcuma Longa* (figure 6).

### Exemple 7 : Poudre de Piper Longum avant fermentation

Le *piper longum* a été utilisé sous forme de poudre, qui contient de la pipérine.

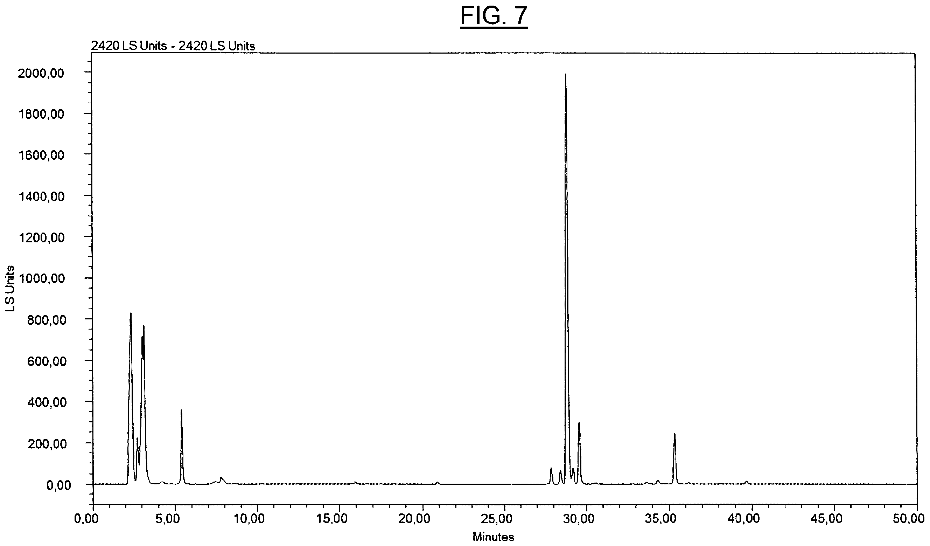

Cette poudre a été analysée puis identifiée par des techniques de type HPLC, HPLC-MS et RMN afin d'obtenir une empreinte chromatographique de l'extrait de *Piper Longum* (figure 7).

### Exemple 8 : Composition contenant des extraits de plantes avant fermentation

Une composition contenant un extrait de *Withania Somnifera* à une concentration de 20 g/L, *d'Emblica Officinalis* à une concentration de 15 g/L, de *Bacopa Monierri* à une concentration de 15 g/L, de *Punica Granatum* à une concentration de 10 g/L, de *Curcuma Longa* à une concentration de 20 g/L, de *Piper Longum* à une concentration de 0,03 g/L, de *Calendula Officinalis* à une concentration de 10 g/L a été préparée à partir de plantes.

Brièvement, on a procédé au broyage des matières végétales. Chaque plante a ainsi été broyée de manière indépendante des autres plantes, à l'aide de broyeurs de type HGB50E-Blender de façon à obtenir des poudres les plus fines possibles, de l'ordre de 80 microns de diamètre.

Puis on a effectué des extractions de chaque extrait de plante par macération. Durant une telle extraction par macération, la température est comprise entre 40°C et 60°C et la pression à 100 bars pendant les deux cycles de 1 heure. Après extraction, les solutions hétérogènes obtenues sont filtrées sur papier filtre, d'une épaisseur de 870um et d'un diamètre de 170 mm. Les solutions obtenues sont réunies, concentrées sous pression réduite à l'aide d'un rotavapor de type BUCHI R-220-SE pour conduire à une solution aqueuse. Ces solutions sont préalablement congelées à-80°C avant d'être déposées dans le lyophilisateur à -55°c sous 0.1mbar (lyophilisateur de type Edwards (Freese Dryer Modulyo) pour conduire à des extraits secs.

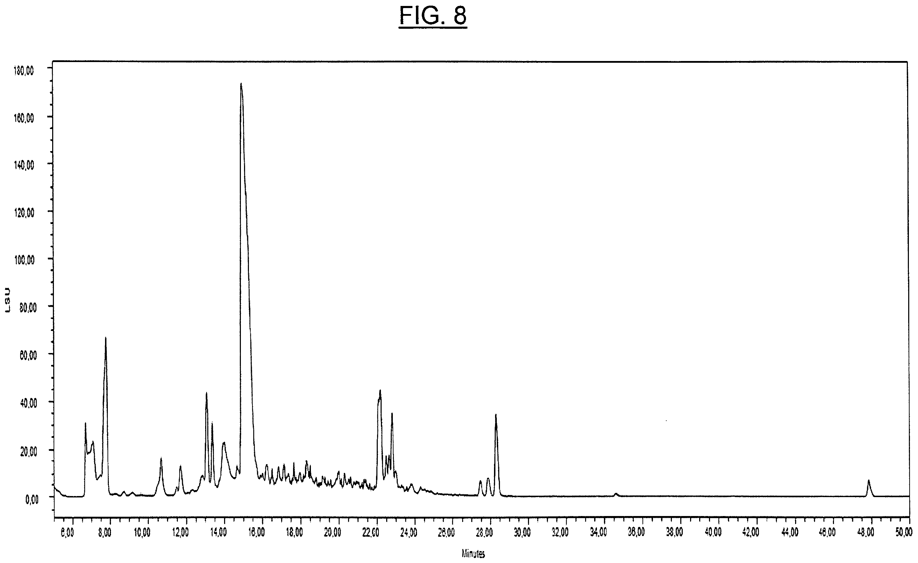

On a ensuite mélangé les différents extraits secs dans un volume d'eau. La solution obtenue est analysée puis identifiée par des techniques de type RMN, HPLC, HPLC-MS afin d'obtenir une empreinte chromatographique illustrée sur la figure 8.

### Exemple 9 : Composition de l'exemple 8 après fermentation

Préalablement à l'incubation, la composition résultant de l'exemple 8 e a été diluée dans de l'eau dans lequel du glucose à une concentration de 50 g/L et du nitrate d'ammonium à une concentration de 20 g/L ont été ajoutés. Cette composition obtenue a ensuite été soumise à une bioconversion par fermentation contrôlée par la souche *Beauveria bassiana* ATCC 7159.

Cette souche a été préalablement cultivée dans un milieu de culture composé de (par litre d'eau) 0,5 g/L KH₂PO₄ ; 1 g/L KH₂PO₄ ; 1 g/L MgSO₄ ; 2 g/L NaNO₃ ; 0,5 g/L KCl ; 0,02 g/L FeSO₄ ; 30 g/L glucose et 10 g/L de liqueur de maïs (Corn steep Liquor, Roquette). La culture est ainsi agitée à 200 rotations par minute, durant 72 heures à 27°C. Elle est ensuite filtrée non stérilement sur un papier filtre pour séparer la biomasse fongique du milieu de culture. La biomasse fongique est ensuite lavée abondamment à l'eau et incubée à 60 g de biomasse fraiche par litre d'incubation qui contient 37.5 g de glucose et 15 g de nitrate d'ammonium.

Après incubation, cette composition ensemencée est placée sous agitation à 200 rpm durant 7 jours à une température de 27°C.

Après 7 jours, le milieu d'incubation est filtré sur un papier, les prélèvements pour analyse HPLC sont également filtrés en filtre de 0,45 microns (Ait-France, ref : SFNY 013045N.

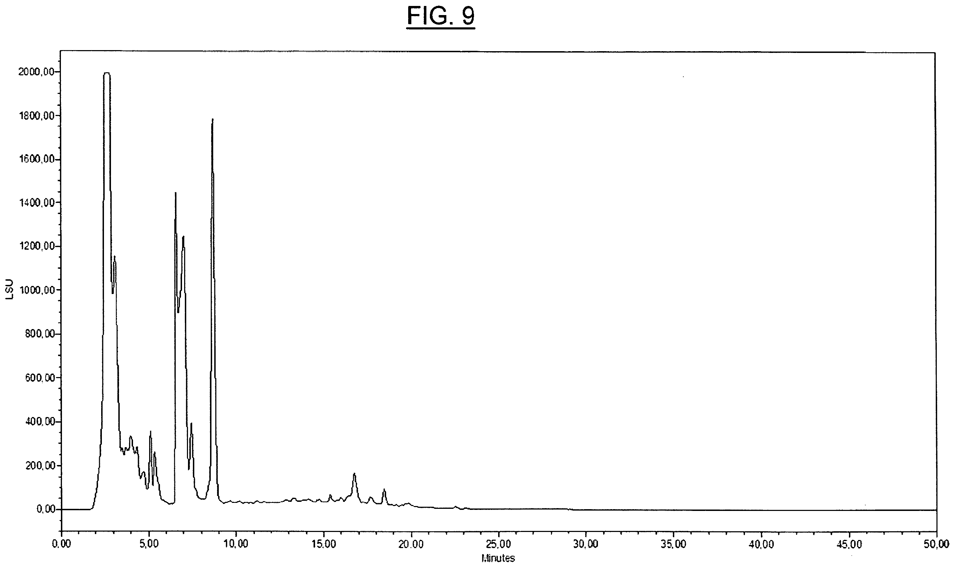

Une solution brunâtre est obtenue, et a été par la suite analysée puis identifiée par des techniques de type RMN, HPLC, HPLC-MS afin d'obtenir une empreinte chromatographique illustrée sur la figure 9.

Les échantillons obtenus ont été analysé comme dans l'exemple 1.

### Exemple 10 : composition selon l'invention avant fermentation

La composition est constituée de *Withania Somnifera* à une concentration de 40 g/L, *d'Emblica Officinalis* à une concentration de 30 g/L, de *Bacopa Monierri* à une concentration de 30 g/L.

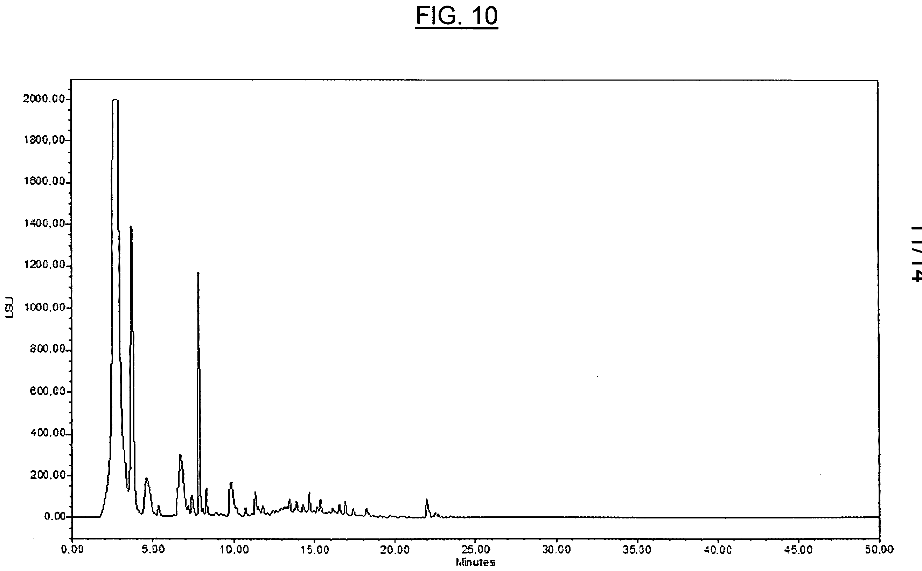

Elle a été analysée par les mêmes techniques que celles de l'exemple précédent (Figure10).

### Exemple 11 : caractérisation d'éléments de la composition selon l'exemple 10

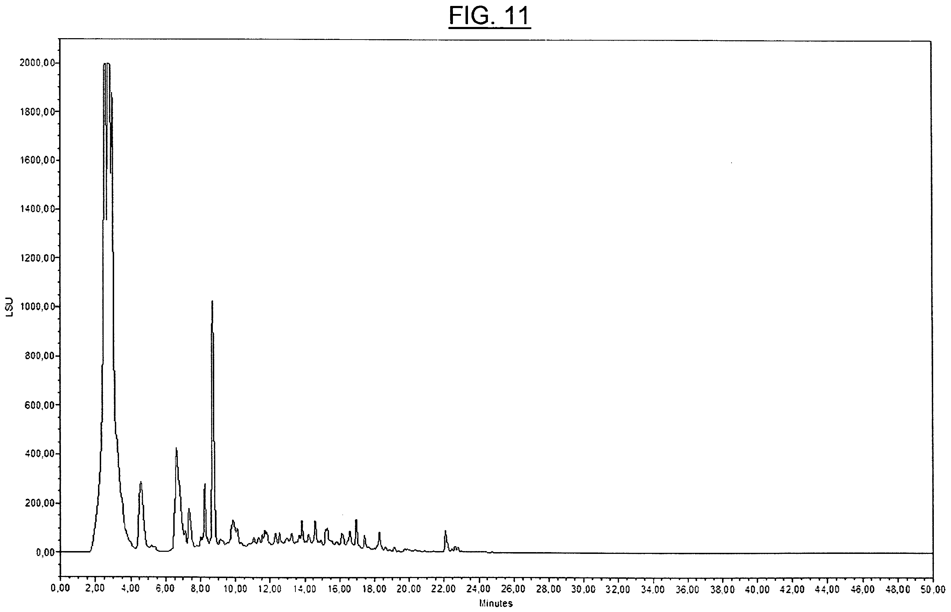

Après l'étape de fermentation, la composition de l'exemple 11 est caractérisée plus précisément (Figure 11).

Le milieu d'incubation est filtré sur un papier, les prélèvements pour analyse HPLC sont également filtrés sur filtres de 0,45 microns (Ait-France, ref : SFNY 013045N) avant injection pour analyse. Les marqueurs identifiés dans la composition sont le Withanoside IV, le Withanoside VI, le Bacoside A3, le Bacopaside X, le Bacopasaponin C, ainsi que l'acide Gallique et son dérivé. 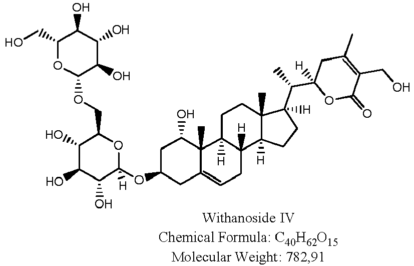 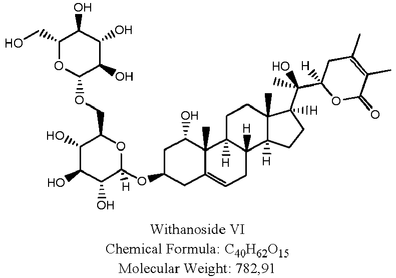 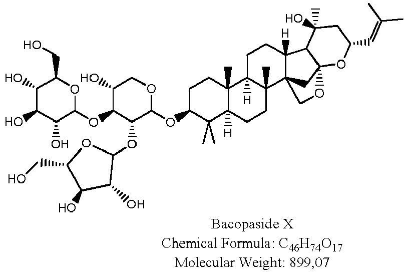 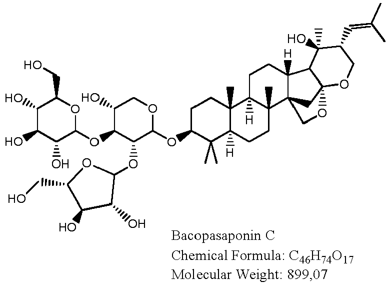 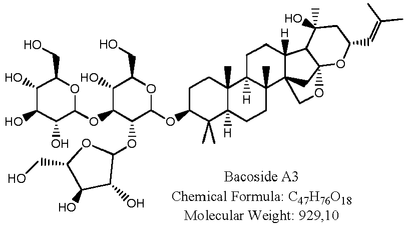 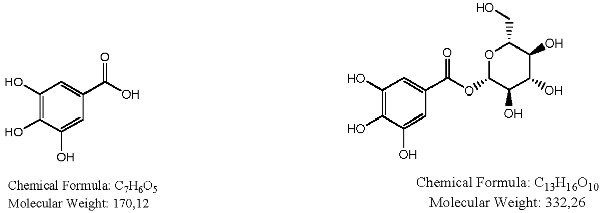 Acide Gallique et son dérivé osidique

### Exemple 12 : composition selon l'invention avant fermentation

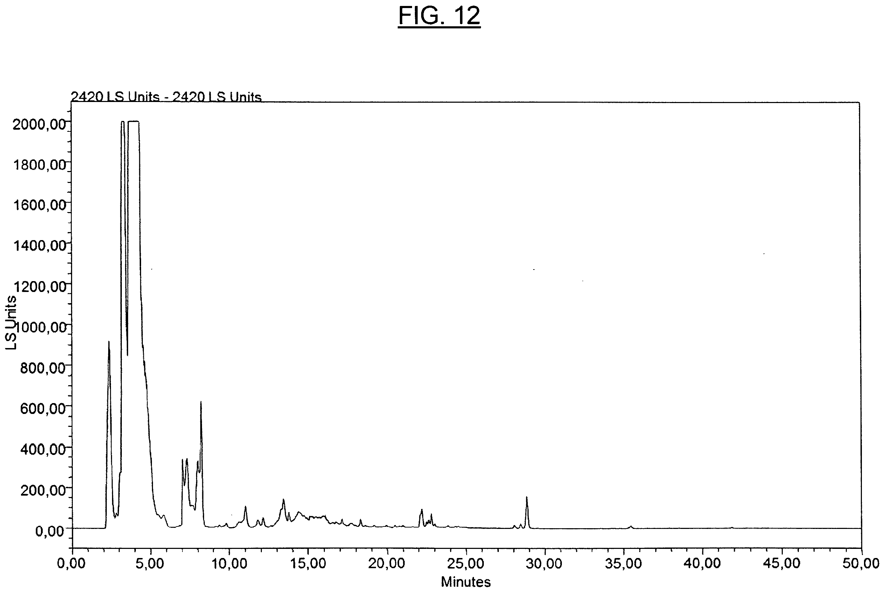

La préparation est constituée de *Withania Somnifera* à une concentration de 20 g/L, *d'Emblica Officinalis* à une concentration de 15 g/L, de *Bacopa Monierri* à une concentration de 15 g/L, de *Piper longum* à une concentration de 20 mg/L, de curcuma à une concentration de 20 g/L (Figure 12).

### Exemple 13 : composition selon l'invention

La préparation est constituée de *Withania Somnifera* à une concentration de 20 g/L, *d'Emblica Officinalis* à une concentration de 15 g/L, de *Bacopa Monierri* à une concentration de 15 g/L (idem figure 10).

### Exemple n°14 : Propriétés anti-angiogéniques des compositions des exemples 8 et 9 (avant et après fermentation)

Des tests biologiques *in ovo,* selon la technique Chicken Chorioallantoic membrane (CAM) ont été réalisés en utilisant la composition de l'exemple 8, de l'exemple 9.

Les oeufs sont fournis par la société EARL les Bruyères, DANGERS. Les œufs utilisés pour les expériences sont livrés le jour de ponte ou au plus tard le lendemain du jour de ponte. Il s'agit d'oeufs issus d'un croisement de poules JA57 et de coqs I66, qui sont normalement destinés à produite des Poussins I657.

Deux jeux de 12 œufs ont été traités 7 jours après fécondation.
- Le premier jeu s'est vu administrer, sur la CAM, 40 µl de la composition de l'exemple 8.
- Le second jeu s'est vu administrer, sur la CAM, 40 µl de la composition de l'exemple 9.

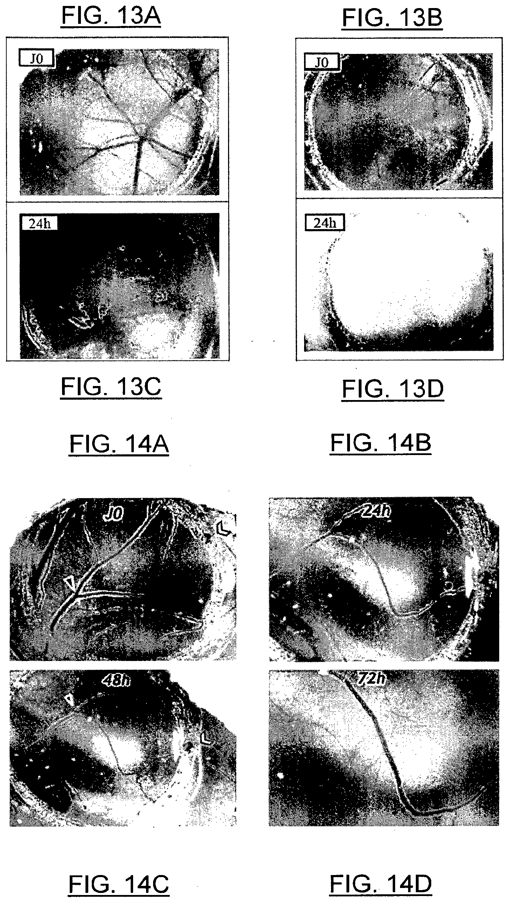

Des photographies de la CAM ont été réalisées avant l'administration, à 24h, 48h et 72h après administration de la composition afin de suivre l'évolution de la vascularisation (figures 14A à 14D).

100% des embryons du premier jeu sont morts dans les 24h (figures 13A à 13D) suite à des hémorragies importantes, vraisemblablement dues à la toxicité de la composition, qui aboutissent à la destruction de la CAM.

Après 78h, seul deux embryons sur les 12 du second jeu sont morts. En outre, la composition de l'exemple 1 fermentée conduit à une très forte réduction du réseau vasculaire chez tous les œufs traités tel qu'observé sur les figures 14A à 14D.

La composition selon l'invention possède donc des propriétés anti-vascularisantes.

De plus, ce test met en évidence la détoxification *in ovo* de la composition obtenue selon le procédé de l'invention.

## Revendications

1. Procédé d'obtention d'un extrait de plante comprenant les étapes suivantes :
- Réalisation d'un extrait *de Withania Somnifera*
- Fermentation dudit extrait par une mise en incubation de cet extrait avec un champignon filamenteux de la famille des *Cordycipitaceae* dans un milieu adapté.

2. Procédé d'obtention d'un extrait selon la revendication 1, dans lequel le champignon de la famille des *Cordycipitaceae* est du genre Beauvaria, et plus particulièrement *beauvaria Bassiana.*

3. Procédé d'obtention d'un extrait selon l'une quelconque des revendications 1 à 2, comprenant, après la fermentation, une étape de filtration du milieu de culture.

4. Procédé d'obtention d'un extrait selon l'une quelconque des revendications 1 à 3, comprenant, après fermentation, une étape de stérilisation du milieu de culture.

5. Procédé d'obtention d'un extrait selon l'une quelconque des revendications 1 à 4, comportant une étape de déshydratation du milieu de culture après fermentation.

6. Procédé d'obtention d'un extrait selon l'une quelconque des revendications 1 à 5, dans lequel, à l'extrait de *Withania Somnifera,* on ajoute un extrait *d'Emblica Officinali* et un extrait de *Bacopa Monnieri* avant de réaliser la fermentation desdits extraits avec ledit champignon filamenteux dans un milieu adapté.

7. Composition comprenant un extrait de la plante *Withania Somnifera* susceptible d'être obtenu par le procédé de l'une quelconque des revendications 1 à 6.

8. Composition comprenant un extrait des plantes *Withania Somnifera, Emblica Officinali* et *Bacopa Monnieri* susceptible d'être obtenu par le procédé de la revendication 6.

9. Composition selon la revendication 7 ou 8, comprenant une quantité en poids de *Withania Somnifera* comprise entre 5 et 100 g/L de *Withania Somnifera,* et de préférence 20g/L.

10. Composition selon l'une quelconque des revendications 7 à 9, comprenant en outre au moins un extrait issu des plantes suivantes *Emblica Officinalis, Bacopa Monnieri, Punica Granatum, Curcuma Longa, Piper Longum,* ou *Calendula Officinalis.*

11. Composition selon l'une quelconque des revendications 7 à 10, **caractérisée par le fait qu'**elle se présente sous forme liquide, gel, émulsion, solide ou injectable.

12. Composition selon l'une quelconque des revendications 7 à 11, en tant que complément alimentaire.

13. Composition selon l'une quelconque des revendications 7 à 11, en tant que médicament.

14. Composition selon la revendication 12, pour son utilisation dans le traitement ou la prévention des désordres liés à une vascularisation dans un mammifère.

15. Composition selon la revendication 12 à 13, pour son utilisation dans le traitement de la DMLA, la rétinopathie liée au diabète, les kératites et les glaucomes associés à une vascularisation anarchique.

16. Composition selon la revendication 12 à 13, pour son utilisation dans le traitement de l'arthrite rhumatoïde, l'arthrose, la maladie de Crohn, l'athérosclérose, les formes d'angiomes de l'enfance ou liés à des maladies héréditaires.

17. Composition selon la revendication 12 à 13, pour son utilisation dans le traitement du cancer, et plus particulièrement des tumeurs solides et des métastases.

18. Composition selon la revendication 12 à 13, pour son utilisation dans le traitement des tumeurs bénignes telles que neurinome de l'acoustique, le neurofibrome, le trachome et des granulomes pyogènes

## Patentansprüche

1. Verfahren zum Erhalten eines Pflanzenextrakts, umfassend die folgenden Schritte:
- Herstellung eines Extrakts von *Withania somnifera*
- Fermentation des Extrakts durch Inkubation des Extrakts mit einem filamentösen Pilz aus der Familie *Cordycipitaceae* in einem geeigneten Medium.

2. Verfahren zum Erhalten eines Extrakts nach Anspruch 1, wobei der Pilz aus der Familie *Cordycipitaceae* aus der Gattung Beauvaria stammt und insbesondere *Beauvaria bassiana* ist.

3. Verfahren zum Erhalten eines Extrakts nach einem der Ansprüche 1 bis 2, umfassend nach der Fermentation einen Schritt der Filtration des Kulturmediums.

4. Verfahren zum Erhalten eines Extrakts nach einem der Ansprüche 1 bis 3, umfassend nach der Fermentation einen Schritt der Sterilisation des Kulturmediums.

5. Verfahren zum Erhalten eines Extrakts nach einem der Ansprüche 1 bis 4, umfassend einen Schritt der Dehydratisierung des Kulturmediums nach der Fermentation.

6. Verfahren zum Erhalten eines Extrakts nach einem der Ansprüche 1 bis 5, wobei man zu dem Extrakt von *Withania somnifera* einen Extrakt von *Emblica officinali* und einen Extrakt von *Bacopa monnieri* hinzugibt, bevor die Fermentation der Extrakte mit dem filamentösen Pilz in einem geeigneten Medium durchgeführt wird.

7. Zusammensetzung, umfassend einen Extrakt der Pflanze *Withania somnifera,* der durch das Verfahren nach einem der Ansprüche 1 bis 6 erhältlich ist.

8. Zusammensetzung, umfassend einen Extrakt der Pflanzen *Withania somnifera, Emblica officinali* und *Bacopa monnieri,* der durch das Verfahren nach Anspruch 6 erhältlich ist.

9. Zusammensetzung nach Anspruch 7 oder 8, umfassend eine Menge an *Withania somnifera,* bezogen auf Gewicht, zwischen 5 und 100 g/l *Withania somnifera* und vorzugsweise 20 g/l.

10. Zusammensetzung nach einem der Ansprüche 7 bis 9, umfassend außerdem mindestens einen Extrakt, der aus den folgenden Pflanzen stammt: *Emblica officinalis, Bacopa monnieri, Punica granatum, Curcuma longa, Piper longum* oder *Calendula officinalis.*

11. Zusammensetzung nach einem der Ansprüche 7 bis 10, **dadurch gekennzeichnet, dass** sie in flüssiger, Gel-, Emulsions-, fester oder injizierbarer Form vorliegt.

12. Zusammensetzung nach einem der Ansprüche 7 bis 11 als Nahrungsergänzungsmittel.

13. Zusammensetzung nach einem der Ansprüche 7 bis 11 als Arzneimittel.

14. Zusammensetzung nach Anspruch 12 zur Verwendung bei der Behandlung oder Vorbeugung von durch Vaskularisierung bedingten Störungen bei einem Säugetier.

15. Zusammensetzung nach Anspruch 12 bis 13 zur Verwendung bei der Behandlung von AMD, diabetischer Retinopathie, durch unkontrollierte Vaskularisierung bedingten Keratiten und Glaukomen.

16. Zusammensetzung nach Anspruch 12 bis 13 zur Verwendung bei der Behandlung von rheumatoider Arthritis, Arthrose, Morbus Crohn, Atherosklerose, Formen infantiler oder durch Erbkrankheiten bedingter Angiome.

17. Zusammensetzung nach Anspruch 12 bis 13 zur Verwendung bei der Behandlung von Krebs und insbesondere soliden Tumoren und Metastasen.

18. Zusammensetzung nach Anspruch 12 bis 13 zur Verwendung bei der Behandlung benigner Tumoren wie Akustikusneurinom, Neurofibrom, Trachom und pyogenen Granulomen.

## Claims

1. Method for obtaining a plant extract, comprising the following steps:
- production of a *Withania somnifera* extract
- fermentation of said extract by incubating this extract with a filamentous fungus of the family *Cordycipitaceae* in a suitable medium.

2. Method for obtaining an extract according to Claim 1, in which the fungus of the family *Cordycipitaceae* is of the *Beauvaria* genus, and more particularly *Beauvaria bassiana.*

3. Method for obtaining an extract according to either one of Claims 1 and 2, comprising, after the fermentation, a step of filtration of the culture medium.

4. Method for obtaining an extract according to any one of Claims 1 to 3, comprising, after fermentation, a step of sterilization of the culture medium.

5. Method for obtaining an extract according to any one of Claims 1 to 4, comprising a step of dehydration of the culture medium after fermentation.

6. Method for obtaining an extract according to any one of Claims 1 to 5, in which an *Emblica officinali* extract and a *Bacopa monnieri* extract are added to the *Withania somnifera* extract before carrying out the fermentation of said extracts with said filamentous fungus in a suitable medium.

7. Composition comprising an extract of the *Withania somnifera* plant that can be obtained by means of the method of any one of Claims 1 to 6.

8. Composition comprising an extract of the *Withania somnifera, Emblica officinali* and *Bacopa monnieri* plants that can be obtained by means of the method of Claim 6.

9. Composition according to Claim 7 or 8, comprising an amount by weight of *Withania somnifera* of between 5 and 100 g/l of *Withania somnifera,* and preferably 20 g/l.

10. Composition according to any one of Claims 7 to 9, also comprising at least one extract from the following plants *Emblica officinalis, Bacopa monnieri, Punica granatum, Curcuma longa, Piper longum* or *Calendula officinalis.*

11. Composition according to any one of Claims 7 to 10, **characterized in that** it is in liquid, gel, emulsion, solid or injectable form.

12. Composition according to any one of Claims 7 to 11, as a food supplement.

13. Composition according to any one of Claims 7 to 11, as a medicament.

14. Composition according to Claim 12, for use thereof in the treatment or prevention of vascularization-related disorders in a mammal.

15. Composition according to Claims 12 to 13, for use thereof in the treatment of ARMD, diabetic retinopathy, keratitis and glaucoma related to anarchic vascularization.

16. Composition according to Claim 12 or 13, for use thereof in the treatment of rheumatoid arthritis, osteoarthritis, Crohn's disease, atherosclerosis, infantile angioma forms or hereditary disease-related angioma forms.

17. Composition according to Claims 12 to 13, for use thereof in the treatment of cancer, more particularly of solid tumours and metastases.

18. Composition according to Claims 12 to 13, for use thereof in the treatment of benign tumours such as acoustic neuroma, neurofibroma, trachoma and pyogenic granulomas.
